# EUROPEAN PATENT SPECIFICATION

(11) **EP 3 413 868 B1**
(45) Date of publication and mention of the grant of the patent: **14.08.2019**
(21) Application number: 16809172.6
(22) Date of filing: 08.11.2016
(51) Int. Cl.: A61K 31/7032, A61K 31/728, A61K 9/00, A61P 27/02, A61K 45/06, A61K 47/12, A61K 9/08, A61K 47/36

(54) **OPHTHALMIC COMPOSITION FOR THE USE IN THE TREATMENT OF EYE DISORDERS RELATED TO ALTERATIONS OF THE CORNEAL CONJUNCTIVAL SURFACE**
OPHTHALMISCHE ZUSAMMENSETZUNG ZUR VERWENDUNG BEI DER BEHANDLUNG VON AUGENERKRANKUNGEN IM ZUSAMMENHANG MIT VERÄNDERUNGEN DER HORNHAUTBINDEHAUTOBERFLÄCHE
COMPOSITION OPHTALMIQUE DESTINÉE À ÊTRE UTILISÉE DANS LE TRAITEMENT DE TROUBLES DE LA VUE ASSOCIÉS À DES ALTÉRATIONS DE LA SURFACE CORNÉENNE-CONJONCTIVALE

(30) Priority: 08.02.2016 IT UB20160543
(43) Date of publication of application: 19.12.2018
(73) Proprietor: Sooft Italia S.p.A., 63833 Montegiorgio (FM) (IT)
(72) Inventor: BIONDI, Marco, 63822 Porto San Giorgio (FM) (IT); BIONDI, Piero, 63822 Porto San Giorgio (FM) (IT); STAGNI, Edoardo, 00165 Roma (IT); STAGNI, Marcello, 63822 Porto San Giorgio (FM) (IT)
(74) Representative: Sarpi, Maurizio
(86) International application number: PCT/IB2016/056719
(87) International publication number: WO 2017/137820

(56) References cited:
- EP-A2- 2 494 954
- RAH ET AL: "A review of hyaluronan and its ophthalmic applications", OPTOMETRY - JOURNAL OF THE AMERICAN OPTOMETRIC ASSOCIATION, ELSEVIER, NL, vol. 82, no. 1, 1 January 2011 (2011-01-01), pages 38-43, XP027573889, ISSN: 1529-1839 [retrieved on 2010-12-23]
- ROBERT L ET AL: "Biological effects of hyaluronan in connective tissues, eye, skin, venous wall. Role in aging", PATHOLOGIE ET BIOLOGIE, L'EXPANSION SCIENTIFIQUE FRANCAISE, PARIS, FR, vol. 58, no. 3, 1 June 2010 (2010-06-01), pages 187-198, XP027063426, ISSN: 0369-8114, DOI: 10.1016/J.PATBIO.2009.09.010 [retrieved on 2009-11-22]

## Description

### Technical field of the invention

The present invention relates to the medical pharmaceutical field and, more specifically, the technical field of topical ophthalmic preparations that can be applied onto the corneal surface and used as a tear substitute, to stabilize and reintegrate the tear film, indicated in case of alterations of the corneal-conjunctival surface due to inflammatory states, oxidative stress, unbalance of the redox potential and accumulation of free radicals, dry eyes and traumatic abrasions. Furthermore, the composition according to the invention has demonstrated to have also anti-inflammatory, antibacterial and re-epithelising activity.

### Background of the invention

Because of its continuous contact with the external environment, the eye represents one of the organs that more easily and frequently, undergoes the insults; in particular cornea and conjunctiva are exposed to the risk of traumatic and damaging events because of their structure, specific function and anatomical position. The cornea is the membrane that covers the front of the eye, through which it is possible seeing the iris and the pupil, it is in direct relation with the outside and protect the internal part of the eye. The front surface of the cornea, convex and directly in relation with the external environment, is coated by the tear film.

The conjunctiva, instead, is the thin membrane that covering the front surface of the eyeball, with the exception of the cornea, and the inner surface of the eyelids. The conjunctiva has the main function of protecting the eye front surface by foreign bodies and infections, furthermore, it facilitates the sliding of the eyelids in the blinking phases and allowing the eyeball to move, without friction of the surfaces, thanks to the secretion of mucin component of the tear film (a kind of viscous mucus that protects the cornea and allows the prominent aqueous layer of the tear film to stratify).

The tear film is the thin layer of liquid distributed on the ocular surface, it has variable thickness depending on its location, with maximum thickness in correspondence of the cornea (about 8 µ). The tear film is composed for 98% of water and for the remaining 2% of proteins and lipids, it is continuously and uniformly distributed on the ocular surface by closing the eyelids. The tear film plays an important function in our sight, as it makes uniform the corneal surface and improves the optical quality of the image. The tear film enables adequate lubrication, reduces friction of the eyelids, allows the transport and the diffusion of molecules and fundamental elements for the epithelial and corneal physiology, such as oxygen, carbon dioxide, ions, mucin and lipids. Furthermore, the tear film performs antibacterial activity, it ensures the molecular turnover and maintain the ocular surface clean by removing the impurities from the environment, metabolism waste products and desquamated cells. Ocular surface and tear film act as a single functional entity. Balancing and integrity of their anatomical components, such as epithelia and lacrimal glands, their innervation and immune response, pH and right concentration of electrolytes and some organic substances, environmental conditions, such as wind, relative humidity and temperature, have direct influence on their physiological functioning.

Tears are produced by lacrimal glands and continuously and uniformly distributed on the conjunctival mucosa and on the cornea by winking, the involuntary closing of the eyelids occurring at least 12-14 times in a minute.

Thus, the tear film has several functions:
- it lubricates the eye, creating on the cornea a smooth surface through which the eyelids can easily scroll,
- it nourishes the cornea, which as devoid of blood vessels receives nutrients and oxygen directly through the tear film, and finally
- it removes waste products and protects the eye from infections.

Furthermore, the eye, because of its particular anatomical structure and its continuous contact with the external environment, certainly represents one of the organs that more easily and frequently, undergoes the insults exercised by oxidative stress, i. e. the disturbance in the balance between the production of reactive oxygen species (free radicals) and antioxidant defence.

Most of the biological processes normally generate free radicals that can even have beneficial effects, but when the formation of free radicals becomes excessive, they can be extremely destructive and attack the fundamental components of cells, such as lipids, proteins and DNA.

In particular, the action of the oxidative stress at corneal level results in the appearance of structural and functional alterations in both the epithelium and corneal stroma associated with the apoptosis in keratinocytes and stromal fibroblasts.

The continuous exposure to visible radiation (photochemical reaction) resulting in the production of reactive forms of oxygen, the presence of hydrogen peroxide level in the aqueous humor, the highest concentration of oxygen in the retina as a result of the high blood flow in this structure, and lipofuscin hyperaccumulation in the epithelium of retinal pigment epithelium able to carry photosensitizing action, are typical factors determining oxidative stress in the eye.

Furthermore, there are diseases affecting the eye which are caused by strong oxidative stress, such as in a non-limitative way, cataract and macular degeneration.

Although cataract is a typical disease of aging, it can also affect less advanced age, but in these cases it is linked to secondary factors (diabetes, inflammatory, congenital and iatrogenic causes).

Also macular degeneration seems to be genetic and environmental related. Smoking is considered a serious dose-dependent risk factor, while less clear are inconsistent correlations with exposure to sunlight, high blood pressure, obesity, cataract surgery.

The onset of inflammatory processes in the eye, or the exposure to various radiation type or to chemical agents, may be directly responsible for the significant increase of the physiological concentration of hydrogen peroxide at corneal level with a consequent increase of reactive oxygen species responsible of oxidative action of the cornea.

The oxidative action of the free radicals in cornea is manifested by the appearance of structural and functional alterations in both the epithelium and in the corneal stroma associated with apoptotic processes involving keratocytes and stromal fibroblasts.

From the above, it is clear the important role of oxidative stress in the onset of structural alterations and functional eye responsible for diseases that can seriously compromise the eye health and quality of life of those who are afflicted.

In order to counteract the consequences of oxidative stress very important is to re-establish the physiological condition of the tear film.

Another type of alteration of corneal conjuctival surface which requires a prompt stabilizing and reintegrating action of the tear film and of the underlying tissue continuity, are corneal excoriations, i.e. lesion of a part of the cornea with partial loss of surface tissue.

The most common excoriations are due to traumatic abrasions caused by foreign bodies in the eye, scratches, breakage of contact lenses, unprotected exposure to ultraviolet (UV), etc., wherein the outer layers of the cornea may be involved or, in more complicated cases, the deeper layers are involved as well.

Foreign body sensation, pain, photophobia, eye redness and abundant tearing are the typical symptoms of corneal abrasion. If, however, the central zone of the cornea is affected there will also be a reduction of the visual quality. Generally, if the excoriation is not too deep it may heal within few days. The therapy varies depending on the extent of the lesion. Generally, the corneal abrasion is treated by instillation of an antibiotic collirium or ointment, followed by a bandage for at least 2-3 days, to allow the corneal tissue to reform.

Therefore, the treatment of the complex and multi-factorial alterations of the corneal-conjunctival surface, even associated with ageing, due to the occurrence of inflammatory states, oxidative stress, unbalance of the redox potential and accumulation of free radicals, dry eyes syndrome and traumatic abrasions, all conditions which sometimes may co-occur and establish together, requires particular care and to have available as complete formulations covering multiple activities.

Marjorie J. Rah describes the usefulness of ophthalmic administration of hyaluronic acid in eye disorders such as dry eye and corneal wound healing due to its anti-inflammatory and anti-oxidant activity (Optometry 2011, 82, 38-43). Surprisingly, it has been found that the ophthalmic composition according to the present invention, comprising the association of lactobionic acid and hyaluronic acid, not only better protect against the hyperosmotic damage, which occurs for example in cases of dry eye, promote fast re-epithelization and wound healing of corneal epithelium, but also has a protective effect against bacterial infections.

In the present invention, there is proposed the use of the association of hyaluronic acid and lactobionic acid in an ophthalmic composition for the treatment of ocular disorders to be used as a tear substitute stabilizing and replacing the tear film, indicated in the case of the corneal-conjunctival surface alterations associated in qualitative and quantitative deficiencies of the tear film due to inflammatory states, unbalance of the redox potential, accumulation of free radicals, stress on the ocular surface, dry eye syndrome, ageing, also associated with bacterial infections.

Furthermore, even unexpectedly said composition has demonstrated to be useful in protecting the eye tissues against infections and promoting wound healing, hence it can be useful in the re-epithelisation of the corneal-conjunctival surface.

### Summary of the invention

It is therefore an object of the present invention an ophthalmic composition comprising the association of hyaluronic acid and lactobionic acid, and its use for the treatment of eye disorders characterized by alterations of the corneal-conjunctival surface due to inflammatory states, unbalance of the redox potential, accumulation of free radicals, stress on the ocular surface, associated with ageing, but also less advanced age according to the appended claims.

Further disclosed is the use of said ophthalmic composition for the reparatory regeneration of the corneal epithelium in case of corneal lesions and microlesions, minor laceration of the cornea or conjunctiva, or eye damage caused by traumatic stress.

### Brief description of the drawings

Figure 1 shows the diagrams of the Visual Analogic Scale (VAS) assessment in the four tested groups: A - right eye of patients aged 18-55 years; B - left eye of patients aged 18-55 years; C - right eye of patients over 55 years old; D - left eye of patients over 55 years old.
Figure 2 shows the diagrams relative to the Frequency of Symptoms assessment in Dry Eye (SANDE) in the four tested groups as above.
Figure 3 shows the diagrams relative to the Severity of Symptoms assessment in Dry Eye (SANDE) in the four tested groups as above.
Figure 4 shows the diagrams of the Schirmer test in the four tested groups as above.
Figure 5 shows the diagrams of the Tear Break Time assessment in the four tested groups as above.
Figure 6 shows the diagrams of the Osmolarity assessment in the four tested groups as above.
Figure 7 shows the diagrams of the Oxford staining in the four tested groups as above.
Figure 8 shows the tear fern images (10X Magnification) in a patient who received the treatment according to the invention.
Figure 9 shows the diagram of the *in vitro* cell proliferation on SIRC cells expressed as % of cell proliferation of the control sample.
Figure 10 shows the diagram of the *in vitro* cell proliferation on SIRC cells expressed as percentage of cell proliferation of the control sample 24 hrs and 48 hrs following the incubation with the tested compositions.
Figure 11 (A) shows the diagram expressing the wound closure percentage *in vitro* on SIRC cells following the incubation with sodium lactobionate, hyaluronic acid and their combinations.
Figure 11 (B) shows the images of wound closure on SIRC cells monolayer in the tested treatments.
Figure 12 (A) shows fluorescein staining micrograph of a representative corneal wound obtained at 0, 24, 48, and 72 h after abrasion. The first row of panels shows injured corneal epithelium treated with PBS as positive control at different time point; the bottom panel shows uninjured corneal epithelium as negative control.
Figure 12 (B) shows the diagram expressing the wound closure percentage *in vivo* on rabbit corneas following the instillation of a composition comprising 4% sodium lactobionate and 4% sodium lactobionate, 0,15% sodium hyaluronate (example 1).
Figure 13 (A) shows the diagram of the variation in matrix metalloproteinase-9 (MMP-9) expression in rabbit tears at 24, 48 and 72 hours during wound healing.
Figure 13 (B) shows the diagram of the variation in matrix metalloproteinase-9 (MMP-9) expression in rabbit corneas at 48 and 72 hours during wound healing.
Figure 14 (A) shows the diagram of the variation TGF-β expression in rabbit tears at 48 hours during wound healing.
Figure 14 (B) shows the diagram of the variation in TGF-β expression in rabbit corneas at 48 and 72 hours during wound healing.

### Description of the invention

The purpose of the present invention is to provide a pharmaceutical composition formulated for ophthalmic topical administration, with a stabilizing and reintegrating action of the tear film, with re-epithelising and wound-healing activity, with antibacterial action, whose fundamental and characteristic component is the association of lactobionic acid, or a salt thereof, and hyaluronic acid, or a salt thereof.

The lactobionic acid is a complex poly-hydroxy acid, also known as aldonic acid of the polysaccharide, is made of a poly-hydroxy acid, gluconic acid and galactose.

Experimental observations indicate that topical application of lactobionic acid increases skin turgidity and corrects the histological signs of skin aging with tolerability and irritation profiles comparable to those of the physiological solution. Furthermore, the poly-hydroxy acids do not cause sensory irritation phenomena and, therefore, are compatible with clinically sensitive skin, including rosacea conditions and atopic dermatitis, and may be employed after cosmetic treatment. The lactobionic acid, as well as most of the poly-hydroxy acids, has strong humectants and moisturizing properties, it stimulates the function of the barrier constituted by the corneal layer of the epidermis, and therefore stimulates the protective function of the stratum corneum, increasing the skin resistance to the chemical insult.

In the Italian patent n. 1404752, granted to the Applicant, for the first time the use of lactobionic acid in an eye drop for the treatment of corneal edema, also in association to inflammation phenomena, has been disclosed. Said eye drops is characterized by the presence of lactobionic acid in a concentration varying from 5% w/w to 20% w/w, and more preferably from 6% w/w to 16% w/w; in addition to lactobionic acid, the solutions according to the invention may further comprise other hyperosmotic agents, such as mannitol or sodium chloride, alone or in combination. Hyaluronic acid is a natural constituent of connective tissue, namely, it is a glycosaminoglycan, or a molecule formed by long, unbranched chains of disaccharide units with alternating glucuronic acid and N-acetylglucosamine.

Hyaluronic acid is used in many fields:
in cosmetics, in the formulation of moisturizing, anti-aging and anti-wrinkle creams;
in aesthetic medicine, in the form of injectable filler to correct minor skin imperfections, such as wrinkles, thin lips, emptied breasts, scarring, etc;
in orthopaedics, hyaluronic acid is injected by intra-articular route (infiltration) for the treatment of arthrosis;
in the healthy field, the molecule is used for the preparation of oral anti-aging supplements; and
in ophthalmology, the hyaluronic acid is used for the preparation of eye drops or ophthalmic ointments for promoting healing of the conjunctiva following traumas.

Ophthalmic ointments and eye drops prepared with hyaluronic acid are useful in the treatment of all ocular disorders characterized by apparent dryness (dry eye or keratoconjunctivitis sicca) or by conjunctival lesions (e.g. traumatic conjunctivitis). In these cases, hyaluronic acid promotes the healing of the conjunctiva in relatively short time and, attenuating the symptoms, keeps the eye hydrated. Object of the present invention is to provide an ophthalmic composition isotonic characterized by the presence of the association of lactobionic acid and of hyaluronic acid, or their respective salts, particularly preferred are their sodium salts. According to the invention in the composition the concentration of lactobionic acid, or a salt thereof, varies from 1% w/w to 5% w/w, preferably from 3.5% w/w to 4.5% w/w, still more preferably the concentration of lactobionic acid is 4.0% w/w.

According to the invention the concentration of hyaluronic acid, or a salt thereof, in the composition varies between 0.01% w/w and 1.0% w/w, preferably between 0.05% w/w and 0.5% w/w, and more preferably the hyaluronic acid concentration is 0.15% w/w. Preferably the hyaluronic acid used in the present invention has a weight between 1 and 2 MDa.

The composition according to the invention is an aqueous solution, clear and colorless, with pH between 7.0 and 7.4, and isotonic osmolality between 250 and 350 mOsm/Kg.

The pH value is due to a buffering system suitable to maintain the pH value in the range between 7.0 and 7.4. Various buffering systems known to the skilled of the field can be employed in some embodiments of the present invention. However, in some preferred embodiments of the present invention the buffering system of sodium phosphate monobasic/dibasic is used. The isotonicity of the composition is ensured by the presence of an adequate amount of lactobionic acid and of hyaluronic acid, or their salts, in particular of their sodium salts.

In addition to the association of lactobionic acid and hyaluronic acid, or their salts, the composition of the present invention may further comprise stabilizing agents of the solution, lubricating agents, moisturizing agents such as hydroxypropylmethylcellulose, or structural or functional analogues.

According to the invention the composition also comprises preservatives and chelating agents. Among the preservatives and chelating agents, particularly preferred are disodium EDTA and N-hydroxymethylglycinate.

According to the invention the ophthalmic composition may include in its formulation also one or more additional therapeutic agents selected from the group of growth factors, anti-inflammatory agents, vitamins, fibronectin, collagen and amino acids.

The formulations of the present invention are prepared by mixing and stirring the ingredients until all components are in solution.

The solution so obtained is then sterilized by filtration (0.2 micron) or by means of heat treatment at temperature of 121 °C, for 20 min, at 1 atmosphere. However, the sterilization conditions may be varied and optimized by the skilled persons.

### Preferred embodiments of the invention

According to the present invention the ophthalmic composition can be prepared in any pharmaceutical form that permits its application in the conjunctival sac, such as eye drops, collirium, gels, ointments, spray, but preferably it is prepared in the form of eye drops, administrable as drops, each drop constituting a form of dosage unit so as to allow easy administration and uniformity of dosage.

For illustrative purposes two examples of particularly preferred formulation are presented.

**Example 1 - Formulation of eye drops**

| Ingredient | % w/w |
|---|---|
| Sodium lactobionate | 4.0 |
| Sodium hyaluronate | 0.15 |
| Sodium dihydrogen phosphate dihydrate | 0.225 |
| Disodium phosphate dehydrate | 0.686 |
| Disodium EDTA | 0.100 |
| Sodium Hydroxymethylglycinate | 0.002 |
| Distilled water | q.s to 100 g |

In a particularly preferred embodiment of the invention, usually 1-2 drops of the composition are instilled into the conjunctival sac, 2-3 times a day or more, however, the therapeutically effective amount is defined by the physician as a function of patient condition. The composition can also be instilled while wearing contact lenses. Dosage and method of administration will be adjusted to provide sufficient levels of the active ingredient or to maintain the desired effect. Additional factors that should be considered include: severity of the condition, course of the disease, age, weight and gender of the patient, time and frequency of administration, diet, association with other drugs, sensitivity reactions, tolerance and response to therapy.

The use of the present invention is not limited to humans, but can be extended, where appropriate, to other mammals.

The collyrium according to the present invention has proved to be highly tolerable by patients, who in clinical tests attested a wellbeing feeling after using the product.

According to the invention the provided composition is particularly simple, as it comprises a very limited number of components and excipients: this represents an advantageous feature that allows to reduce to a minimum the risk of possible allergic or sensitization phenomena.

In particular, in the embodiment of the single-dose formulation or preservative-free multi-dose formulation, the preservative system can be eliminated.

**Example 2 - Formulation of the single-dose or preservative-free multi-dose eye drops**

| Ingredient | % w/w |
|---|---|
| Sodium lactobionate | 4.0 |
| Sodium hyaluronate | 0.15 |
| Sodium dihydrogen phosphate dihydrate | 0.225 |
| Disodium phosphate dihydrate | 0.686 |
| Distilled water | q.s. to 100 g |

According to the results of the experimental studies carried out, a particularly interesting field of application for the composition according to the invention is the treatment of alterations of the corneal-conjunctival surface due to inflammatory states, oxidative stress, unbalance of the redox potential and accumulation of free radicals, dry eyes syndrome and traumatic abrasions. Furthermore, the experimental results have demonstrated that the invention provide an ophthalmic composition for the use in wound-healing and re-epithelisation of the corneal-conjunctival surface.

In fact, the data from the study described below relating the analysis of protein markers involved in the wound healing process phenotypically confirmed what is known from the literature. It is known that both MMP-9 and TGF-β correlate negatively with the healing process of wounds; particularly in wound healing processes affecting the cornea, high levels of both proteins, over time, delay the mechanisms implicated in corneal re-epithelization [Kim E.C. et al. Acta Ophthalmol. 2012. 90: 540-6]. The levels of both proteins are reduced in tears and in corneas, 48 hours after injury, following the instillation of the composition having formulation as described in example 1, comprising 4% sodium lactobionate and 0.15% sodium hyaluronate, provide experimental evidence of a rapid re-epithelizating process of the cornea following the use of said composition.

Surprisingly, the ophthalmic composition according to the invention has demonstrated to also provide protection against bacterial. Further investigation are currently on going, preliminary data in relation to this activity are encouraging.

Because of its further technical features the use of the invention is particularly important because it avoids the use of bacteriostatics, like antibiotics and chemical disinfectants, which are too aggressive and irritant for the eye, preferring substances nontoxic, well tolerated, whose safety is well confirmed. The composition according to the invention having antibacterial activity, re-epithelising activity can be used to counteract the alterations of the corneal-conjunctival surface due to inflammatory states, unbalance of the redox potential, accumulation of free radicals, and oxidative stress.

The composition object of the invention thanks to its components adds to the action of tear substitute stabilizing and replenish the tear film, common to many similar products already available on the market, also re-epithelising, wound healing and antibacterial action

The invention and its accomplishment will be now described through examples which will enable the skilled persons to fully understand the advantages deriving from the use thereof.

### EXPERIMENTAL PART

### Study 1 - Assessment of safety and efficacy of the association of lactobionic acid and hyaluronic acid in the treatment of dry eye syndrome

The study is designed to assess the safety and the efficacy of a eye drop composition containing lactobionic acid, or a salt thereof, and hyaluronic acid, or a salt thereof, compared to an identical composition not containing lactobionic acid, against a set of symptoms typically recognized in the eye dry syndrome. In the study five-weeks treatment phase was followed by one week follow-up period to evaluate safety of the composition.

At day 7, day 21, day 35, day 45 multiple ophthalmologic assessments were performed in order to exclude adverse effect of the tested compositions on the eye structure and physiology.

Visual Analogic Scale (VAS). A global ocular discomfort score was determined using a 100 mm visual analogue scale (VAS) wherein 0 means no symptom and 100 means indicates the worse possible discomfort. This evaluation was performed before any ophthalmic assessment at any visit. Specific ocular symptoms to be assessed with the VAS included: foreing body sensation, burning/stinging, itching, pain, sticky feeling, blurred vision, photophobia.

Symptom Assessment iN Dry Eye (SANDE). Since there is no "gold standard" diagnostic test for DED (Dry Eye Disease), a combination of signs and symptoms are commonly used as diagnostic criteria. Because of the variability of dry eye symptoms and the limitations of the available clinical tests, questionnaires recording patients' symptoms are very useful tools for diagnosis and follow-up of dry eye disease. The Symptom Assessment Questionnaire iN Dry Eye (SANDE) is a short and intuitive questionnaire based on a visual analog scale that quantifies both severity and frequency of dry eye symptoms. The SANDE is comprised of two questions, and each question employs a 100 mm horizontal linear visual analog scale. The measurement of symptom frequency ranges from "rarely" to "all of the time," and the symptom severity from "very mild" to "very severe."

Schirmer test is a simple test enabling to quantify the basal tear secretion, which results to be decreased in some diseases (SLE, rheumatoid arthritis, Sjogren's Sindrome) or due to iatrogenic causes related to taking medication. It is performed by placing 2 mm paper tissue strips in the conjunctival fornix and wait a few minutes (5 min). Then the portion of moistened strip is measured; below a certain value is classified as dry eye, so a replacement therapy is necessary.

Eye is normal when ≥15 mm wet paper is obtained after 5 min; eye is mild dry when 14-9 mm wet paper is obtained after 5 min; eye is moderately dry when 8-4 mm wet paper is obtained after 5 min; eye is severely dry when <4 mm wet paper is obtained after 5 min.

Tear breakup time (TBUT) is a clinical test used to assess for evaporative dry eye disease. To measure TBUT, fluorescein is instilled into the patient's tear film and the patient is asked not to blink while the tear film is observed under a broad beam of cobalt blue illumination. The TBUT is recorded as the number of seconds that elapse between the last blink and the appearance of the first dry spot in the tear film, as seen in this progression of these slit lamps photos over time. A TBUT below 10 seconds is considered abnormal.

Osmolarity Change in Tear Film Osmolarity has been evaluated by TearLab Instrument. Osmolarity < 290 mOsm is considered normal, >290<316 is considered mild; >316 is moderate to severe.

Slit lamp examination (SLE) following Oxford staining with fluoroscein to evaluate alteration in eyelid margin, conjunctiva, cornea, anterior chamber, iris and lens. Some degree of corneal staining is a normal finding in up to 79% of corneas and 71% of conjunctivae in normal eyes. However, signs of corneal staining are also indicative of compromised cornea. Interestingly, it is still not absolutely clear how fluorescein acts on the ocular surface, however, it is commonly accepted that it penetrates only discontinuities or gaps in the epithelium and into intracellular spaces and underlying tissues via damaged cells.

### Inclusion criteria:

In the clinical study have been included:
- Male and female adults aged 18 years and over;
- Subjects with at least 3-months documented history of dry eye in both eyes diagnosed as dry eye syndrome, keratoconjunctivitis sicca (KCS) or due to Sjogren syndrome;
- Subjects having discontinued all artificial tears through the duration of the treatment period;
- Subjects who have not used steroid eye drops during 4 weeks prior the study;
- Subjects having discontinued lens wear one week prior the study and during the entire study.

From the clinical study have been excluded:
- Women in pregnancy or lactation;
- Potential childbearing women who are not using systemic contraception, not postmenopausal, not surgically sterilized;
- Subjects undergone to any type of ocular surgery or ocular trauma within four months prior the study;
- Subjects affected by other diseases or condition judged by the investigator/physician to be incompatible with the assessment needed in this study;
- Subjects having ongoing active inflammation of the eye different from KCS;
- Participation to any other clinical trial within 30 days prior the study.

Patients enrolled in the study have been divided in two groups according to the age: Group A, 20 patients aged between 18 and 50 years and Group B, 20 patients older than 50 years. Within each group randomly half of the subjects received the composition comprising the association of 4% sodium lactobionate and 0.15% sodium hyaluronate acid and half received the control composition comprising only 0.15% sodium hyaluronate. All patients received 1-2 drops of composition three times a day for five weeks followed by one week follow-up period.

### Results

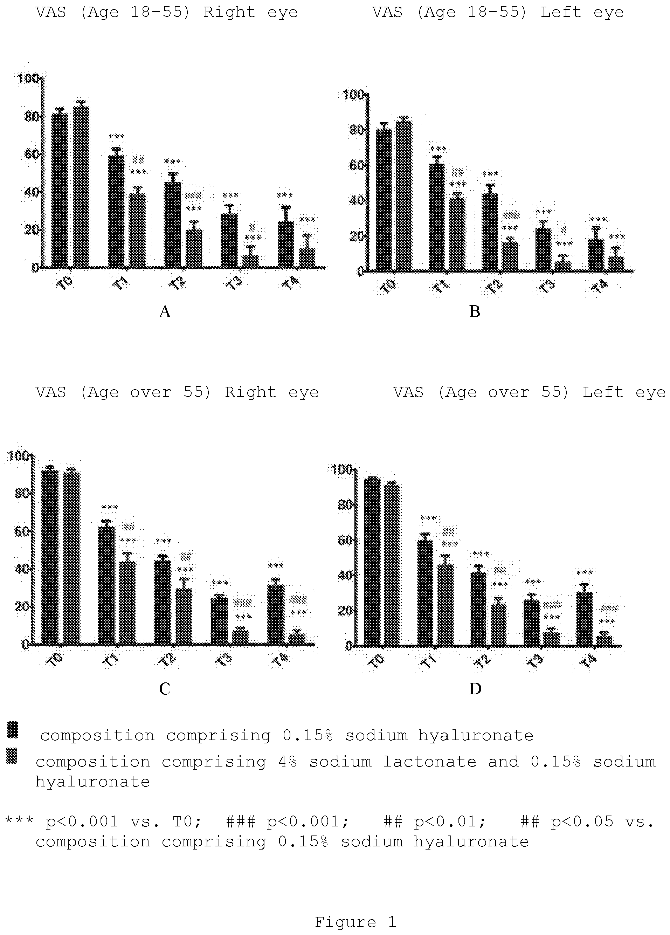

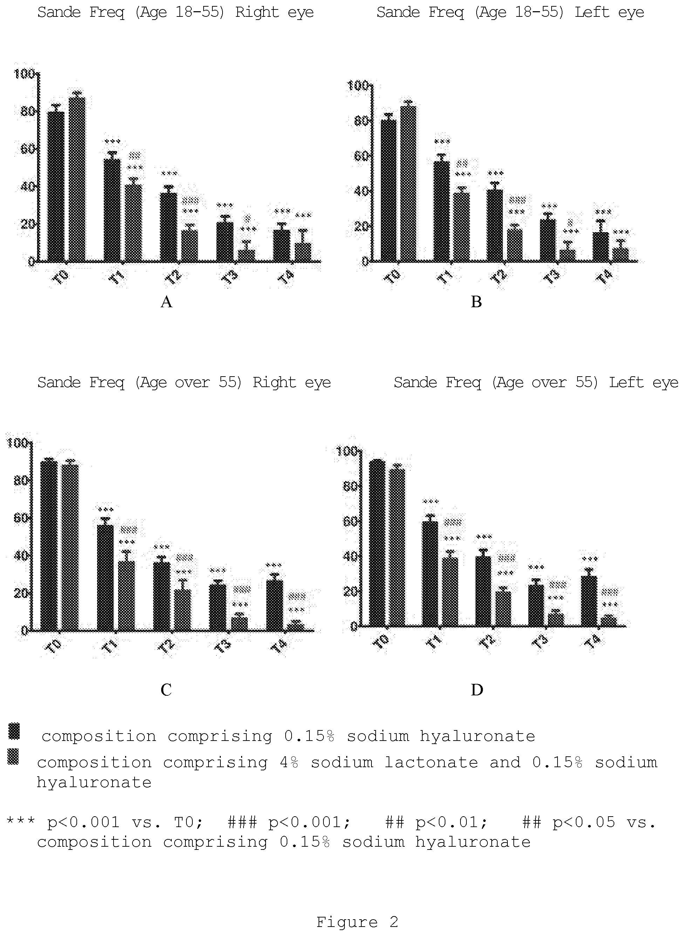

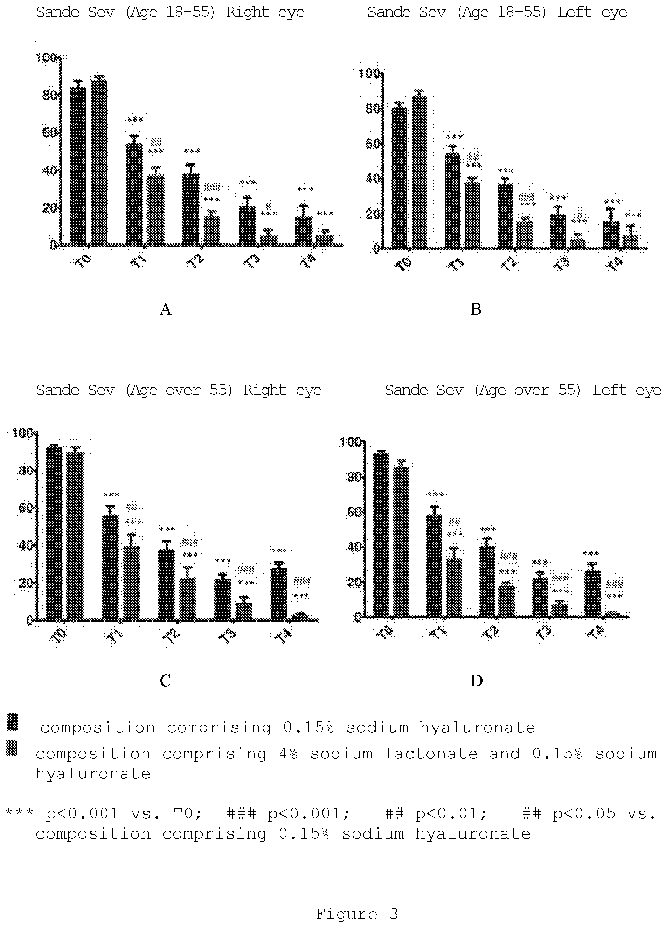

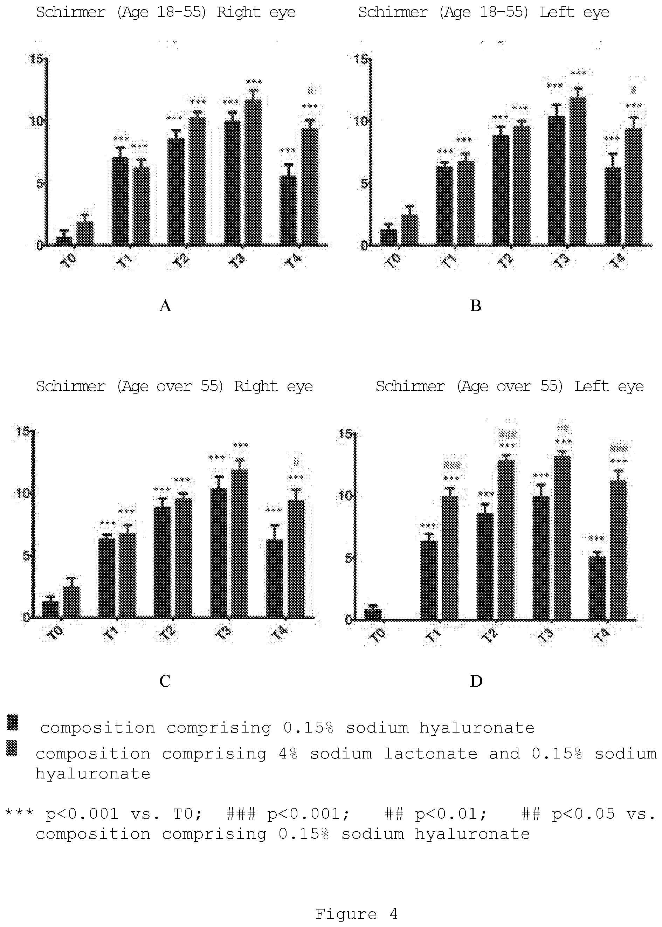

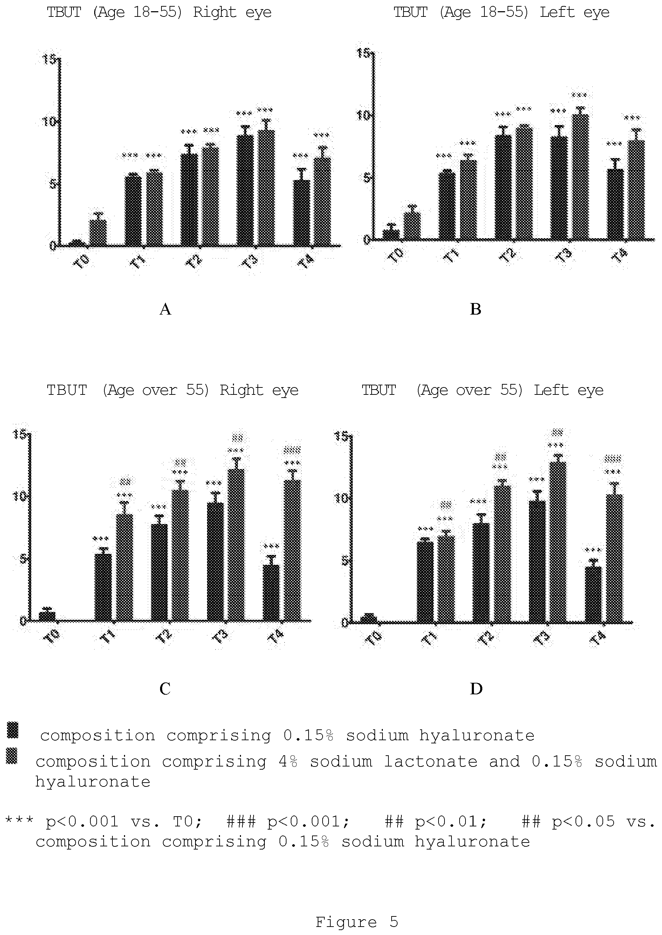

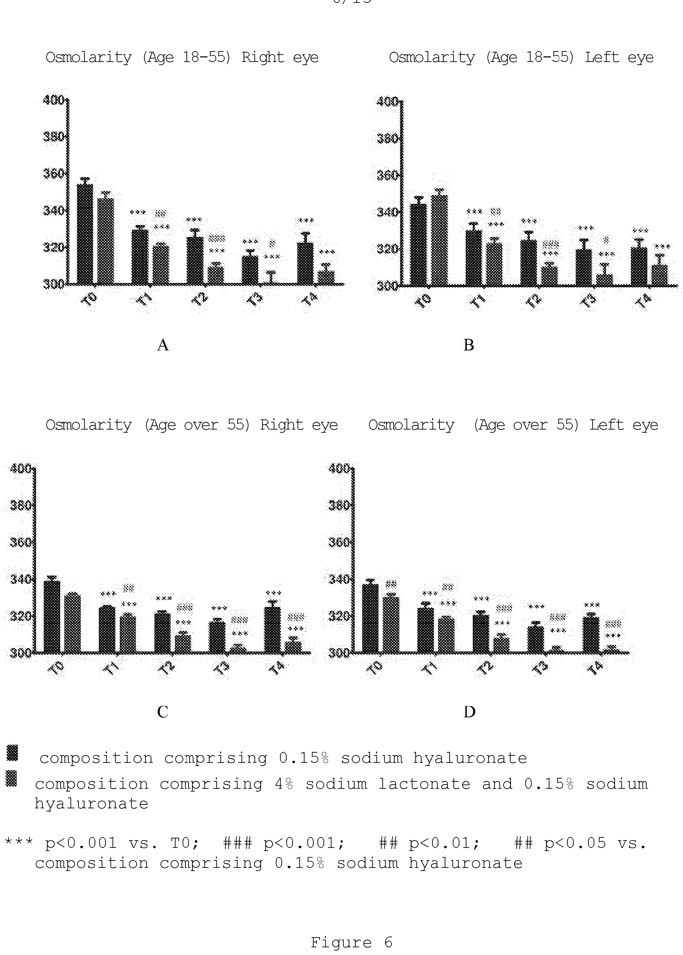

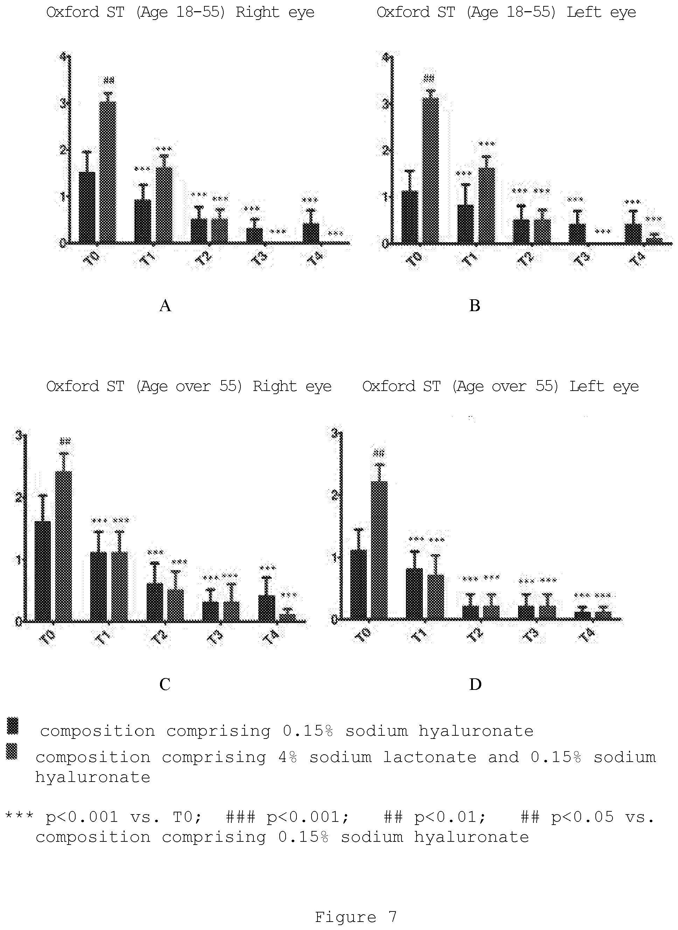

The results of the assessment of safety and efficacy of the association of lactobionic acid and hyaluronic acid in the treatment of dry eye syndrome have been shown in figures 1-7 by the evaluation of the above mentioned parameters and demonstrate that eye drops based on hyaluronic acid alone or the association of hyaluronic and lactobionic acid are both effective - as expected - in improving symptoms and signs of dry eye already after a week of treatment. However, patients treated with the association of hyaluronic and lactobionic acid showed a significant better improvement also with respect to patients treated with hyaluronic acid alone, and such difference was more evident in the group of patients over 55 years of age, thus indicating a superiority of the association of hyaluronic and lactobionic acid, especially in the advanced age. No other eye drops are known to have this different behaviour with respect to the age of patients.

### Study 2 - Tear ferning test

Practical insight into tear film composition is very useful to the clinician for patient diagnosis and treatment. The tear ferning test is a laboratory test, but it can be applied in the clinic setting to investigate the tear film composition in a simple way. It consists in drying a small sample of tear fluid onto a clean, glass microscope slide; this produces a characteristic crystallisation pattern, described as a 'tear fern'.

The ferning test provides a qualitative indication of the tear film stability in an indirect way: so the test can assess the balance between the mucoproteins and the salts dissolved in tears and then the osmolarity of the tear film.

In our study the patient (female, 40 years old, affected by dry eye) has received the treatment with the composition according to the invention for 15 minutes.

### Results

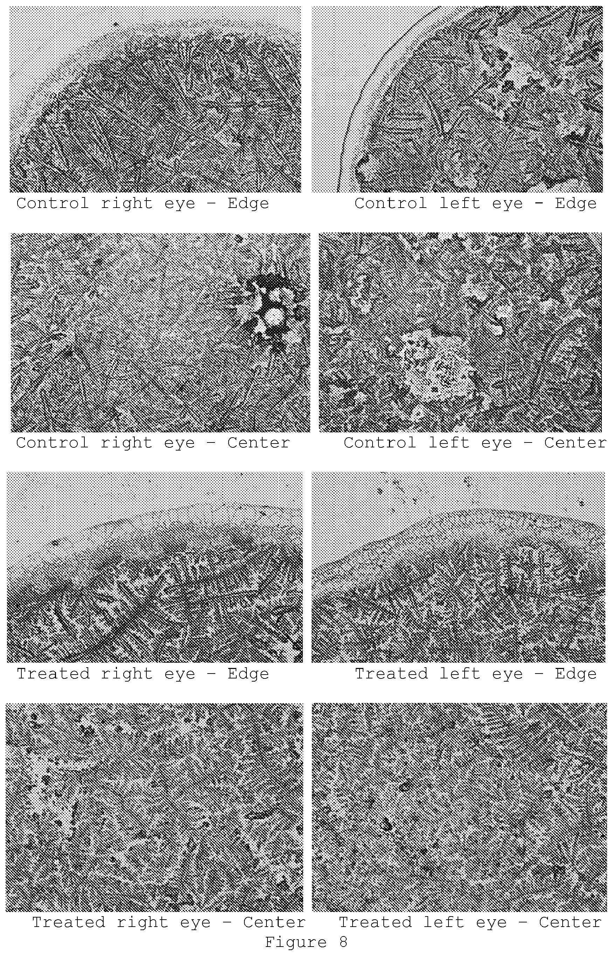

Results of the ferning test are shown in Figure 8, the image (10x magnification) of the obtained crystallisation pattern indicates that before treatment ferning was very poor at the tear borders and incomplete in the tear center. Fifteen minutes after Lactoyal administration a denser and more structured ferning pattern could be observed both at the tear borders, and at the center of the tear.

### Study 3 - Crystal Violet Assay for Determining effect of the composition according to the invention on survival and growth inhibition of cultured cell

Adherent cells detach from cell culture plates during cell death. This characteristic can be used for the indirect quantification of cell death and to determine differences in proliferation upon any stimulation by detecting adherent and viable cells stained with crystal violet dye binding to proteins and DNA. Cells that undergo cell death lose their adherence and are subsequently lost from the population of cells, reducing the amount of crystal violet staining in a culture. This assay protocol provide a quick and reliable screening method suitable to evaluate the impact of a composition on cell survival and growth inhibition. Upon solubilization, the amount of dye taken up by the cell monolayer can be quantified in a spectrophotometer or plate reader.

SIRC cells [Statens Seruminstitut Rabbit Cornea] (ATCC® CCL-60™) were seeded and grown up to the desired density according to manufacturer's protocol.

Assayed compositions are:
CTRL = control (PBS, Phosphate buffered saline);
LS 1% = 1% lactobionate sodium;
HA 0.03% = 0.03% sodium hyaluronate;
HA 0.075% = 0.075% sodium hyaluronate;
MIX 1 = 1% lactobionate sodium, 0.03% sodium hyaluronate;
MIX 2 = 1% lactobionate sodium, 0.075% sodium hyaluronate;
After washing with PBS crystal violet solution was added to the cells and incubated for 10 minutes at room temperature.

Carefully culture medium was removed from wells.

Plate was gently washed with PBS warmed at least to room temperature.

Then the plate was washed twice in tap water by immersion in a large beaker and drained upside down on paper towels. To solubilize the stain, 1% SDS was added to each well.

Plate was agitated on orbital shaker until color is uniform with no areas of dense coloration in bottom of wells.

Absorbance of each well was read by spectrophotometer at 570 nm.

### Results

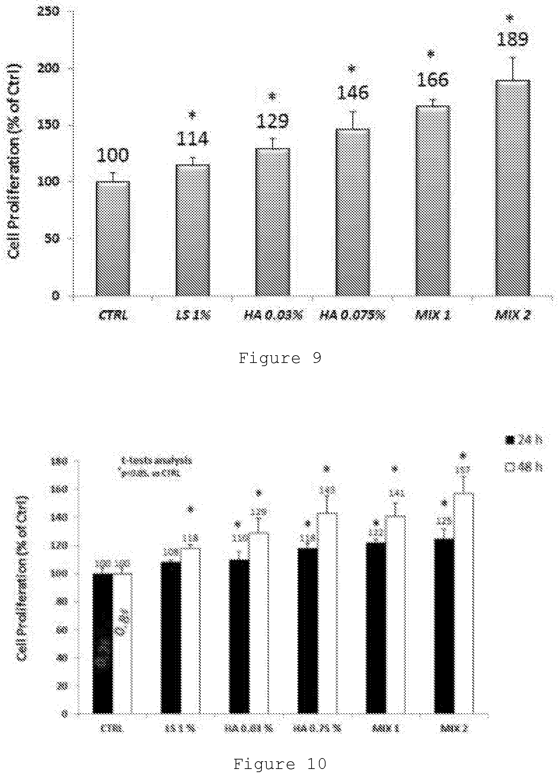

The cell proliferation values obtained following the treatment with the assayed compositions are shown in the diagrams of Figure 9 wherein is evident the synergic effect of sodium lactobionate and sodium hyaluronate in the composition according to the invention.

Figure 10 shows the cell proliferation values after 24 hours and 48 hours of incubation with the tested compositions.

### Study 3 - Wound healing assay on SIRC cells

The direct biological consequence of the administration of the composition comprising sodium lactobionate and sodium hyaluronate on SIRC cells was investigated by cell migration assay, i.e. migration of SIRC cell to wound closure. SIRC cells were incubated in a 24-well multiwell plates, coated by fibronectin and type I collagen, using MEM with 10% (v/v) fetal bovine serum until the growth fully covered the well surface. A cell lifter was used to scratch a line across the center of the dish. The remaining cells on both sides were washed twice with PBS. Then, cells were incubated for 24, 48 and 72 hrs at 37°C with 5% CO2 respectively with:
1. medium (MEM with 10% [v/v] fetal bovine serum) (CTRL);
2. medium (MEM with 10% [v/v] fetal bovine serum) containing 1% sodium lactobionate (LS 1%);
3. medium (MEM with 10% [v/v] fetal bovine serum) containing 0.03% sodium hyaluronate (HA 0.03%);
4. medium (MEM with 10% [v/v] fetal bovine serum) containing 0.075% sodium hyaluronate (HA 0.075%);
5. medium (MEM with 10% [v/v] fetal bovine serum) containing 1% lactobionate sodium, 0.03% sodium hyaluronate (MIX 1);
6. medium (MEM with 10% [v/v] fetal bovine serum) containing 1% lactobionate sodium, 0.075% sodium hyaluronate (MIX 2).

The extent of migration was photographed and estimated with three separate experiments (triplicate).

### Results

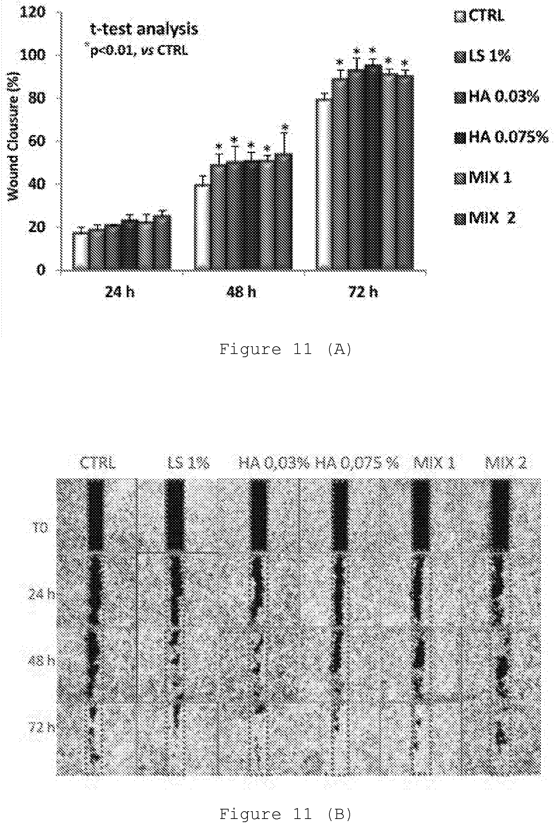

The migration assay was employed as an *in vitro* model for epithelial cell migration during wound healing to examine the influence of sodium lactobionate, sodium hyaluronate, and their combination, on SIRC cells. Results are shown in Figure 11 (A and B). The evidence of cell migration was observed when the scratch in the cell monolayer gradually closed during incubation with sodium lactobionate, sodium hyaluronate, and their combination, for 24 -72 hrs.

The obtained wound closure percentage values on SIRC cells are reported in table 1 and represented in the diagram of Figure 11 (A).

**Table 1**

| | Control CRTL | LS 1% | HA 0.03% | HA 0.075% | MIX 1 | MIX 2 |
|---|---|---|---|---|---|---|
| 24 hrs | 17 | 19 | 21 | 22.8 | 22 | 24.8 |
| 48 hrs | 39.3 | 49 | 50.3 | 50.5 | 50.6 | 53.8 |
| 72 hrs | 79 | 89 | 93 | 95 | 91 | 90 |

Table 1 and Figure 11 (A) show that SIRC cells in all treatment groups proliferated at 24, 48 and 72 hrs compared to those in the control group. Data indicate that variation in wound closure percentage is greater following the incubation for 24 and 48 hrs in the presence of lactobionate sodium and sodium hyaluronate following the incubation with the two single compounds for the same time respect to the control, and such wound closure percentage is even greater when in the association the concentration of sodium hyaluronate is 0.075 % rather than 0.03%. At 72 hour the wound closure appears to be greater due to incubation with only sodium hyaluronate. Hence, said data at 24 and 48 hrs clearly indicate that the association of lactobionate sodium and sodium hyaluronate is able to promote the re-epithelization more rapidly than the single ingredients providing a major proliferative push in a shorter time. The importance of this feature will be evident to the skilled in the field, as it positively interferes with the velocity of wound healing process. At longer incubation time (72 hours) the association ceases his positive action as the re-epithelization process has occurred and completed already. Indeed, it is of evidence the importance that re-epithelization process establish very rapidly, as re-epithelization velocity is limiting the healing outcome and the association of lactobionate sodium and sodium hyaluronate has demonstrated to enable such velocity *in vitro.*

The evidence of cell migration can be observed on the the scratch in the cell monolayer gradually closed during incubation in the pictures of Figure 11 (B).

### Study 4 - Animal study

Male New Zealand white rabbits weighing around 2.5 kg were used in this study. All rabbits were provided by Harlan-Envigo (Milano) and the Ethical Committee of the University of Catania approved all the procedures. Animals were anesthetized with an intramuscular injection of Zoletil (Virbac lab.) at 5 mg/kg (0.1 ml per rabbit). To evaluate the re-epithelizing effect of the composition according to the invention having the formulation as described in Example 1 of the present application and compare it with that of a solution comprising 4% sodium lactobionate, 23 rabbit eyes were subjected to corneal de-epithelialization in one eye by Algerbrush II (Accutome, USA), the contralateral eye was not damaged and used as a control (no wound healing).

Animals were divided into three groups:
- n. 8 rabbit eyes instilled by composition comprising 4% sodium lactobionate;
- n. 7 rabbit eyes instilled by composition of example 1;
- n. 8 rabbit eyes instilled by PBS;

According to the treatment protocol the rabbit eyes received eye drops four time a day every 2 hours. The injured eyes were observed daily (T0, T24, T48, T72) after 1 mg/mL fluorescein staining to demarcate the wounded area. 48 and 72 hours after injury rabbits were sacrificed.

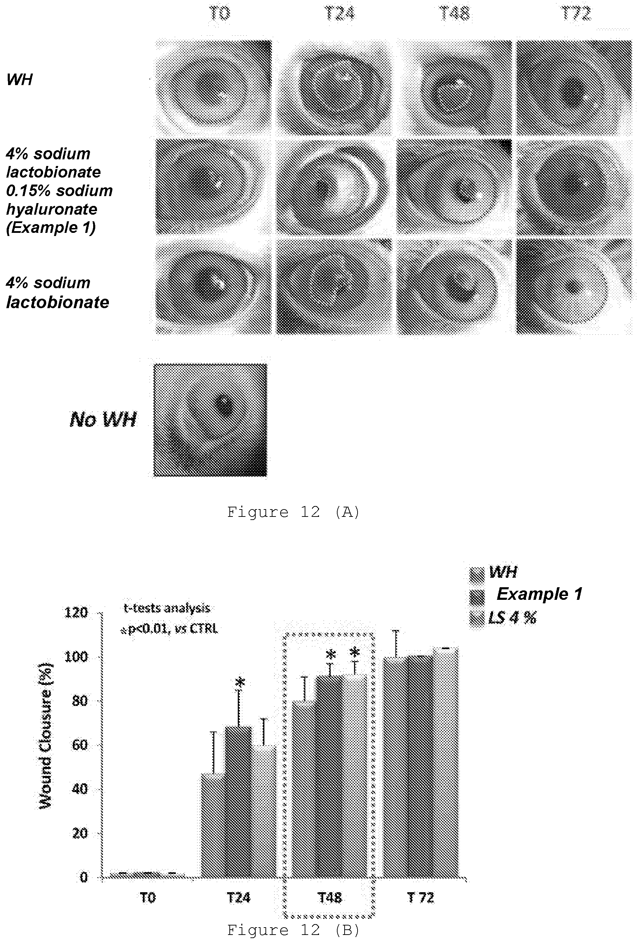

A fluorescein sodium solution was used to evaluate the healing ratio of the corneal epithelium. The area of the corneal wound was quantified from photographs by using Image J analysis software Figure 12 (A). The healing rate was expressed as wound closure percentage. Data are reported in table 2 and graphically presented in diagram of Figure 12 (B).

**Table 2**

| ± dS | Control | Composition comprising 4% sodium lactobionate, 0.15 sodium hyaluronate | Composition comprising 4% sodium lactobionate, |
|---|---|---|---|
| T0 | 0 | 0 | 0 |
| T24 | 47 ± 19 | 68 ± 17 p<0.01 vs control | 60 ± 12 |
| T48 | 80 ± 11 | 91 ± 6 p<0.01 vs control | 92 ± 6 p<0.01 vs control |
| T72 | 100 ± 12 | 100 ± 0.5 | 104 ± 0 |

### Study 5 - MMP-9 activity

There is a large amount of evidence regarding matrix metalloproteinases (MMPs), which play a key role in regulating inflammation, degrading, and remodelling the new ECM (extracellular matrix) during the wound-healing process. As part of the tissue response to injury, the activity of MMPs is strictly regulated at various levels, including gene expression and the tissue inhibitors of MMPs (TIMPs) [Robert A.R. Tetracyclines and the treatment of corneal stromal ulceration: a review. Cornea. 2000;19:274-277. Fini M. E. et al. Proteolytic mechanisms in corneal ulceration and repair. Arch Dermatol Res. 1998; 290 Suppl: S12-S23. Toriseva M. et al. Proteinases in cutaneous wound healing. Cell Mol Life Sci. 2009; 66:203-224]. All classes of extracellular matrix proteins and signaling molecules such as cytokines and growth factors are substrates for the MMPs. In particular, MMP-9, plays a significant role in the remodeling of the corneal stromal ECM and the reformation of the epithelial basement membrane [Mulholland B. et al. Matrix metalloproteinase distribution during early corneal wound healing. Eye. 2005; 19: 584-588. Ollivier F.J. et al. Proteinases of the cornea and preocular tear film. Vet Ophthalmol. 2007; 10: 199-206]. MMP-9, also known as gelatinase B, is secreted by corneal epithelial cells, macrophages and neutrophils during corneal wound healing. The changes in level of MMPs in the tear film and expression of MMP in the cornea during wound healing have been described. MMP-9, the matrix-degrading enzyme, is highly expressed at the site of inflammation and involved in remodeling processes. It also facilitates the recruitment of inflammatory cells, such as eosinophils and neutrophils across basement membranes. It is known that in damaged corneas, proteolysis in the tear fluid has been found to be significantly increased compared to normal eyes.

In our study, change in tear and cornea MMP-9 proteins was evaluated by ELISA according to standard procedures known to the skilled in the field.

### Results

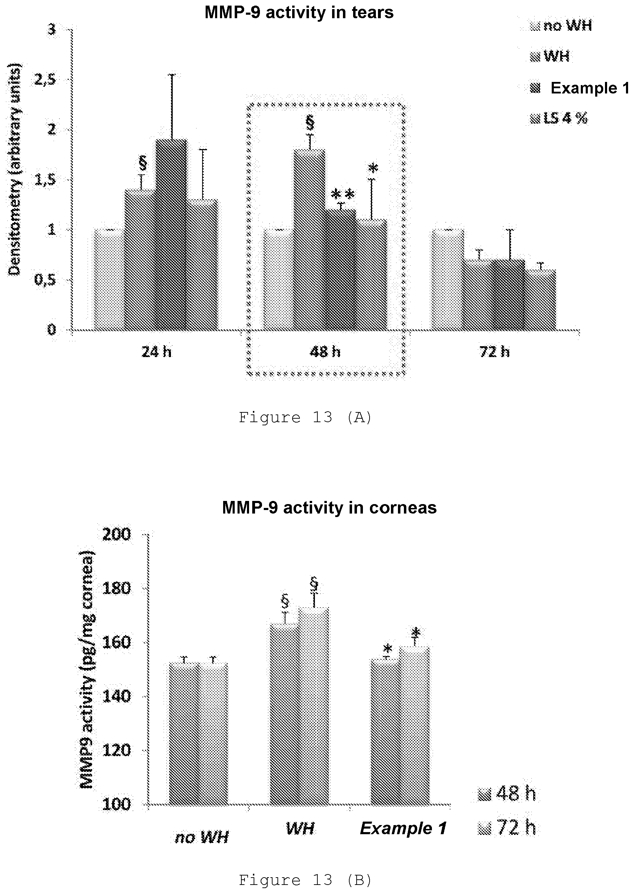

The result of analysis of the MMP-9 activities from the tear samples in the experimental group compared to the control group is shown in table 3 and in the diagram of Figure 13 (A); they displayed a significant decrease in MMP-9 activity in our experimental conditions.

**Table 3**

| ± dS | 24 hrs | 48 hrs | 72 hrs |
|---|---|---|---|
| no WH | 1 ± 0.19 | 1 ± 0.3 | 1 ± 0.01 |
| WH | 1.4 ± 0.15 | 1.8 ± 0.14 | 0.7 ± 0.1 |
| | p<0.01 vs no WH | p<0.01 vs no WH | |
| Composition comprising 4% sodium lactobionate, 0.15% sodium hyaluronate | 1.9 ± 0.75 | 1.2 ± 0.06 | 0.7 ± 0.3 |
| | | p<0.01 vs WH | |
| Composition comprising 4% sodium lactobionate | 1.3 ± 0.5 | 1.1 ± 0.4 | 0.6 ± 0.07 |
| | | p<0.05 vs WH | |

The acceleration of corneal wound healing following the administration of the association of sodium lactobionate and sodium hyaluronate is probably correlated to the reduction in MMP-9 activity and attenuating the inflammation at the wound site.

By ELISA assay it was evaluated the level of metalloproteinases MMP-9 in the cornea of the eyes treated with the composition as formulated in Example 1 of this description. The assay showed that in the treated corneas the activity of the metalloproteinases is reduced compared to that of the positive control which received cornea injury (WH) and alike to the negative control provided by the not injured cornea, as shown in Table 4 and in the diagram Fig. 13 (B).

**Table 4**

| ± dS | 48 hrs | 72 hrs |
|---|---|---|
| no WH | 152 ± 2.1 | 152 ± 2.1 |
| WH | 167 ± 4.2 | 173 ± 5.2 |
| | p<0.01 vs no WH | p<0.01 vs no WH |
| Composition comprising 4% sodium lactobionate, 0.15% sodium hyaluronate | 153 ± 1.1 | 158 ± 3.2 |
| | p<0.01 vs WH | p<0.01 vs WH |

### Study 6 - TGF-β activity

Transforming growth factor-beta (TGF-β) is a multifunctional peptide that modulates cell proliferation and differentiation in many cell types and accelerates tissue repair response. From the literature it is known that in rat corneas 4 hours post-abrasion, acute inflammatory cells showing high positivity to TGF-β appears in the peripheral stroma and gradually spread to the central cornea. By 24 hr, these cells accumulated and formed a pseudomembrane in the epithelial defect, which also showed an intense positivity to TGF-β, suggesting that the peptide participates even in the acute inflammatory response. Usually re-epithelialization occurs over the pseudomembrane and is completed by 48 hours. [Hayashi K. et al. Expression of transforming growth factor-beta in wound healing of vitamin A-deficient rat corneas. Invest Ophthalmol Vis Sci. 1989; 30: 239-47]. In this study, expression of TGF-β was investigated immunohistochemically in the healing of epithelial wounds of corneas of rabbit, both in corneas and in the tears.

On the basis of the results previously obtained showing that the time point critical for the skin restoring effect is 48 hours post-abrasion, the ELISA assay was conducted to measure the level of TGF-β.

### Results

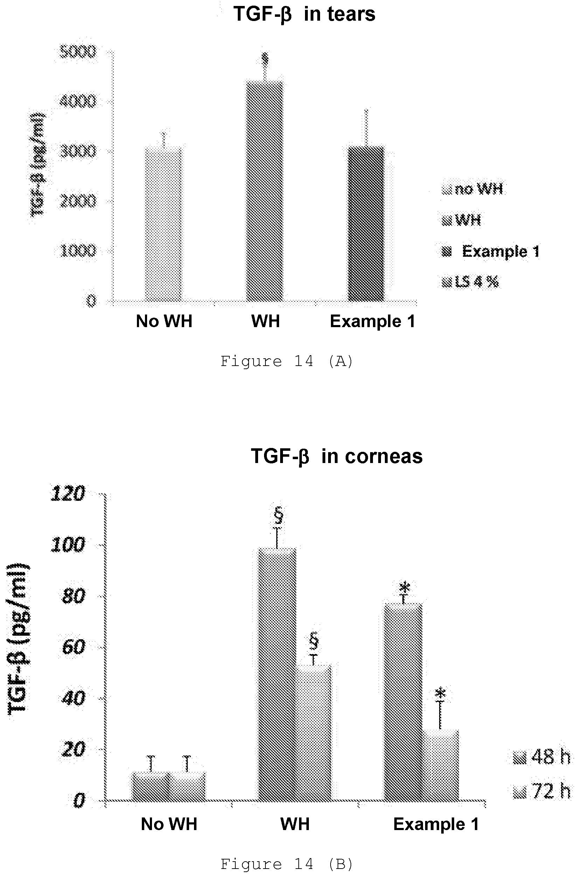

The results showed that in the tear from the eye instilled with the composition having the formulation as in Example 1 the protein level of TGF-β is reduced compared to that of the control eyes (WH) and alike to the not-injured eye. See results in Table 5 and Figure 14 (A).

**Table 5**

| | |
|---|---|
| ± dS | 48 hrs |
| no WH | 3075 ± 288 |
| WH | 4402 ± 308 |
| | p<0.05 vs no WH |
| Composition comprising 4% sodium lactobionate, 0.15% sodium hyaluronate | 3082 ± 743 |

A similar result concerning the TGF-β level has been confirmed also in cornea wherein ELISA assay has highlighted reduced level of TGF-β in corneas of eye treated with the composition comprising 4% sodium lactobionate and 0.15 sodium hyaluronate 48 hours and 72 hours post abrasion. The result confirms a reduced inflammatory response to tissue injury and subsequent wound healing in treated corneas compared to untreated corneas. See results in Table 6 and Figure 14 (B).

**Table 6**

| | | |
|---|---|---|
| ± dS | 48 hrs | 72 hrs |
| no WH | 11,4 ± 6 | 11,4 ± 6 |
| WH | 98,7 ± 8 | 53 ± 4 |
| | p<0.01 vs no WH | p<0.01 vs no WH |
| Composition comprising 4% sodium lactobionate, 0.15% sodium hyaluronate | 77 ± 3.7 | 28 ± 11 |
| | p<0.01 vs WH | p<0.01 vs WH |

### Statistical analysis

All the results were expressed as the mean ± standard deviation (SD). Data were compared between treatment groups using one-way Analysis of Variance (ANOVA). Differences were considered significant at P < 0.05.

### Conclusion

A healthy tear film and healthy cornea and conjunctiva surface are very important for many major functions of the eye. Eye inflammations, post-injury or due to microbiological infections, and dry eye disease are significant clinical problems that needs to be solved, but their complexity and the poor correlation between clinical signs and the diversification of the reported symptoms makes difficult for the clinician to individuate the correct therapeutic approach. Our study demonstrates that the topical administration of a composition comprising lactobionic acid, or a salt thereof, and hyaluronic acid, or a salt thereof, leads to a significantly better response to injury, oxidative stress, ageing. We were able to demonstrate that the pharmaceutical preparation according to the invention rapidly accelerates the healing process while reducing local inflammation. The study substantiates the potential application of a composition comprising sodium lactobionate and sodium hyaluronate for the treatment of superficial corneal injuries and inflammations. Moreover, topical administration seems to be responsible for an increase in cellular epithelialization processes that offers promising scenarios using the association of lactobionic acid and hyaluronic acid or their salts, for therapeutic purposes.

## Claims

1. Ophthalmic composition comprising the association of lactobionic acid, or a salt thereof, ranging between 1% w/w and 5% w/w, and hyaluronic acid, or a salt thereof, ranging between 0.01% w/w and 1.0% w/w, and one or more pharmaceutically acceptable excipients and carriers.

2. Ophthalmic composition according to claim 1, wherein the amount of lactobionic acid, or of its salt, ranges between 3.5% w/w to 4.5% w/w, and the amount of hyaluronic acid, or of its salt, varies between 0.05% w/w and 0.5% w/w.

3. Ophthalmic composition according to any one of the preceding claims, wherein the lactobionic acid, or its salt, is equal to 4% w/w, and hyaluronic acid, or its salt, is equal to 0.15% w/w.

4. Ophthalmic composition according to any one of the preceding claims wherein the salt is a sodium salt.

5. Ophthalmic composition according to any one of the preceding claims **characterized in that** it is isotonic, with osmolality between 250 and 350 mOsm/Kg and having pH between 7.0 and 7.4.

6. Ophthalmic composition according to any one of the preceding claims **characterized in that** it further comprises stabilizing agents of the solution, lubricating agents, moisturizing agents, buffering agents, preservatives and chelating agents.

7. Ophthalmic composition according to claim 6 comprising disodium EDTA and N-hydroxymethylglycinate.

8. Ophthalmic composition according to any one of the preceding claims further comprising additional therapeutic agents, selected from the group of: growth factors, anti-inflammatory agents, vitamins, fibronectin, collagen and amino acids.

9. Ophthalmic composition according to any one of the preceding claims in any form for topical application.

10. Ophthalmic composition according to claim 9 in the form of eye drops, gel, ointment, spray.

11. Ophthalmic composition according to any one of the preceding claims having the following formulation:
| | |
|---|---|
| Lactobionate sodium | 4.0% w/w |
| Sodium hyaluronate | 0.15% w/w |
| Sodium dihydrogen phosphate dihydrate | 0.225% w/w |
| Disodium phosphate dihydrate | 0.686% w/w |
| Disodium EDTA | 0.100% w/w |
| Sodium Hydroxymethylglycinate | 0.002% w/w |
| distilled water | q.s. to 100 g |

12. Ophthalmic composition according to any one of the preceding claims having the following formulation:
| | |
|---|---|
| Lactobionate sodium | 4.0% w/w |
| Sodium hyaluronate | 0.15% w/w |
| Sodium dihydrogen phosphate dihydrate | 0.225% w/w |
| Disodium phosphate dihydrate | 0.686% w/w |
| distilled water | q.s. to 100 g. |

13. Ophthalmic composition according to claims 1 to 12 for use in the treatment of eye disorders **characterized by** inflammatory states, alteration of tear film, dry eye syndrome, oxidative stress, stress on the ocular surface, eye ageing.

14. Ophthalmic composition according to claims 1 to 12 for use in the wound healing of the corneal-conjunctival epithelium.

## Patentansprüche

1. Ophthalmische Zusammensetzung, umfassend die Verbindung von Lactobionsäure oder einem Salz davon, im Bereich zwischen 1 Gew.-% und 5 Gew.-% und Hyaluronsäure oder einem Salz davon, im Bereich zwischen 0,01 Gew.-% und 1,0 Gew.-%, und einem oder mehreren pharmazeutisch annehmbaren Arzneimittelträgern und Trägersubstanzen.

2. Ophthalmische Zusammensetzung nach Anspruch 1, wobei der Anteil von Lactobionsäure oder von ihrem Salz im Bereich zwischen 3,5 Gew.-% bis 4,5 Gew.-% liegt, und der Anteil von Hyaluronsäure oder von ihrem Salz zwischen 0,05 Gew.-% und 0,5 Gew.-% schwankt.

3. Ophthalmische Zusammensetzung nach einem der vorhergehenden Ansprüche, wobei die Lactobionsäure oder ihr Salz 4 Gew.-% entspricht, und Hyaluronsäure oder ihr Salz 0,15 Gew.-% entspricht.

4. Ophthalmische Zusammensetzung nach einem der vorhergehenden Ansprüche, wobei das Salz ein Natriumsalz ist.

5. Ophthalmische Zusammensetzung nach einem der vorhergehenden Ansprüche, **dadurch gekennzeichnet, dass** sie isotonisch mit einer Osmolalität zwischen 250 und 350 mOsm/kg ist und einen pH-Wert zwischen 7,0 und 7,4 besitzt.

6. Ophthalmische Zusammensetzung nach einem der vorhergehenden Ansprüche, **dadurch gekennzeichnet, dass** sie des Weiteren stabilisierende Wirkstoffe der Lösung, Gleitmittel, Feuchthaltemittel, Pufferwirkstoffe, Konservierungsmittel und Komplexbildner umfasst.

7. Ophthalmische Zusammensetzung nach Anspruch 6, umfassend Dinatrium-EDTA (Ethylendiamintetraessigsäure) und N-Hydroxymethylglycinat.

8. Ophthalmische Zusammensetzung nach einem der vorhergehenden Ansprüche, des Weiteren umfassend zusätzliche therapeutische Wirkstoffe, die ausgewählt sind aus der Gruppe: Wachstumsfaktoren, entzündungshemmende Mittel, Vitamine, Fibronectin, Collagen und Aminosäuren.

9. Ophthalmische Zusammensetzung nach einem der vorhergehenden Ansprüche in beliebiger Form zur aktuellen Anwendung.

10. Ophthalmische Zusammensetzung nach Anspruch 9, in Form von Augentropfen, Gel, Salbe, Spray.

11. Ophthalmische Zusammensetzung nach einem der vorhergehenden Ansprüche mit der folgenden Zubereitung:
| | |
|---|---|
| Lactobionatnatrium | 4,0 Gew.-% |
| Natriumhyaluronat | 0,15 Gew.-% |
| Natriumdihydrogenphosphatdihydrat | 0,225 Gew.-% |
| Dinatriumphosphatdihydrat | 0,686 Gew.-% |
| Dinatrium EDTA | 0,100 Gew.-% |
| Natriumhydroxymethylglycinat | 0,002 Gew.-% |
| destilliertes Wasser | so viel wie nötig, bis 100 g |

12. Ophthalmische Zusammensetzung nach einem der vorhergehenden Ansprüche mit der folgenden Zubereitung:
| | |
|---|---|
| Lactobionatnatrium | 4,0 Gew.-% |
| Natriumhyaluronat | 0,15 Gew.-% |
| Natriumdihydrogenphosphatdihydrat | 0,225 Gew.-% |
| Dinatriumphosphatdihydrat | 0,686 Gew.-% |
| destilliertes Wasser | so viel wie nötig, bis 100 g |

13. Ophthalmische Zusammensetzung nach Anspruch 1 bis 12 zur Verwendung bei der Behandlung von Augenerkrankungen, **gekennzeichnet durch** entzündliche Zustände, krankhafte Veränderung des Tränenfilms, Syndrom der trockenen Augen, durch Oxidation entstandene Belastung, Druck auf der Augenoberfläche, Älterwerden der Augen.

14. Ophthalmische Zusammensetzung nach Anspruch 1 bis 12 zur Verwendung bei der Wundheilung des Hornhaut- und Bindehautepithels.

## Revendications

1. Composition ophtalmique comprenant l'association d'acide lactobionique, ou d'un sel de celui-ci, variant entre 1 % p/p et 5 % p/p, et d'acide hyaluronique, ou d'un sel de celui-ci, variant entre 0,01 % p/p et 1,0 % p/p, et un ou plusieurs excipients et supports pharmaceutiquement acceptables.

2. Composition ophtalmique selon la revendication 1, dans laquelle la quantité d'acide lactobionique, ou de son sel, varie entre 3,5 % p/p et 4,5 % p/p, et la quantité d'acide hyaluronique, ou de son sel, varie entre 0,05 % p/p et 0,5 % p/p.

3. Composition ophtalmique selon l'une quelconque des revendications précédentes, dans laquelle l'acide lactobionique, ou son sel, est égal à 4 % p/p, et l'acide hyaluronique, ou son sel, est égal à 0,15 % p/p.

4. Composition ophtalmique selon l'une quelconque des revendications précédentes, dans laquelle le sel est un sel de sodium.

5. Composition ophtalmique selon l'une quelconque des revendications précédentes, **caractérisée en ce qu'**elle est isotonique, avec une osmolalité entre 250 et 350 mOsm/Kg et ayant un pH entre 7,0 et 7,4.

6. Composition ophtalmique selon l'une quelconque des revendications précédentes, **caractérisée en ce qu'**elle comprend en outre des agents stabilisateurs de la solution, des agents lubrifiants, des agents hydratants, des agents tampons, des conservateurs et des agents chélatants.

7. Composition ophtalmique selon la revendication 6, comprenant de l'EDTA dissodique et du glycinate de N-hydroxyméthyle.

8. Composition ophtalmique selon l'une quelconque des revendications précédentes, comprenant en outre des agents thérapeutiques additionnels, choisis dans le groupe des : facteurs de croissance, agents anti-inflammatoires, vitamines, fibronectine, collagène et acides aminés.

9. Composition ophtalmique selon l'une quelconque des revendications précédentes, sous toute forme pour une application topique.

10. Composition ophtalmique selon la revendication 9, sous la forme de gouttes, d'un gel, d'une pommade, ou d'un spray pour les yeux.

11. Composition ophtalmique selon l'une quelconque des revendications précédentes, de formulation suivante :
| | |
|---|---|
| Lactobionate de sodium | 4,0 % p/p |
| Hyaluronate de sodium | 0,15 % p/p |
| Dihydrogénophosphate de sodium dihydraté | 0,225 % p/p |
| Phosphate dissodique dihydraté | 0,686 % p/p |
| EDTA dissodique | 0,100 % p/p |
| Hydroxyméthylglycinate de sodium | 0,002 % p/p |
| Eau distillée | q.s.p. 100 g. |

12. Composition ophtalmique selon l'une quelconque des revendications précédentes, de formulation suivante :
| | |
|---|---|
| Lactobionate de sodium | 4,0 % p/p |
| Hyaluronate de sodium | 0,15 % p/p |
| Dihydrogénophosphate de sodium dihydraté | 0,225 % p/p |
| Phospate dissodique dihydraté | 0,686 % p/p |
| Eau distillée | q.s.p. 100 g. |

13. Composition ophtalmique selon les revendications 1 à 12, à utiliser dans le traitement de troubles des yeux **caractérisée par** des états inflammatoires, une altération du film lacrymal, le syndrome de l'oeil sec, un stress oxydatif, un stress sur la surface oculaire, le vieillissement des yeux.

14. Composition ophtalmique selon les revendications 1 à 12, à utiliser dans la cicatrisation de plaie de l'épithélium cornéen-conjonctival.
